# EUROPÄISCHE PATENTSCHRIFT

(11) **EP 0 957 176 B1**
(45) Veröffentlichungstag und Bekanntmachung des Hinweises auf die Patenterteilung: **19.07.2006**
(21) Anmeldenummer: 99101714.6
(22) Anmeldetag: 10.02.1999
(51) Int. Cl.: C12Q 1/68, G02B 21/00

(54) **Verfahren zum Nachweis von Änderungen in Biopolymeren**
Method for the detection of modifications in biopolymers
Méthode pour la détection des modifications en biopolymères

(30) Priorität: 16.02.1998 DE 19806303; 03.11.1998 DE 19850661
(43) Veröffentlichungstag der Anmeldung: 17.11.1999
(60) Teilanmeldung: 05028091.6
(73) Patentinhaber: MetaSystems Hard & Software GmbH, 68804 Altlussheim (DE)
(72) Erfinder: Chudoba, Ilse, Dr., 68804 Altlussheim (DE); Loerch, Thomas, Dr., 68799 Reilingen (DE); Plesch, Andreas, Dr., 68723 Schwetzingen (DE); Senger, Gabriele, Dr., 93055 Regensburg (DE)
(74) Vertreter: Müller-Boré & Partner Patentanwälte

(56) Entgegenhaltungen:
- WO-A1-97/40191
- SPEICHER, M.R. et al. Karyo- typing human chromosomes by combinatorial multi-fluor FISH. Nature Genetics, Januar 1996, Band 12, Nr. 1, Seiten 368-375, XP002900459, speziell die Seiten 368-370, 374.
- SCHRÖCK, E. et al. Multi- color Spectral Karyotyping of Human Chromosomes. Science, 26. Juli 1996, Band 273, Seiten 494-497, XP002900460, ganzes Dokument.
- PINKEL, D. et al. Cytogenetic analysis using quantitative, high-sensitivity, fluor- escence hybridization. Pro- ceedings of the National Academy of Sciences, Mai 1986, Band 83, Nr. 9, Seiten 2934-2938, XP002900461, speziell die Zusammenfassung, Seite 2938.

## Beschreibung

Die vorliegende Erfindung betrifft ein Verfahren zum Nachweis von Änderungen in Biopolymeren, insbesondere in chromosomaler DNA, unter Verwendung von zwei oder mehreren unterschiedlichen Sätzen von markierten Detektormolekülen sowie einen diagnostischen Kit zum Nachweis dieser Änderungen.

Die Darstellung menschlicher Chromosomen wird bisher mit Bänderungstechniken durchgeführt, die eine spezifische Erkennung der Chromosomen anhand von hellen und dunklen Banden erlauben (z.B. "G-banding, Q-banding, R-handing"). Diese Bänderungstechniken basieren auf Verfahren, die von Caspersson et al. (Exp. Cell Res. 60, 1970, 315-319), Sumner et al. (Nature 232, 1971, 31), Seabright et al. (Lancet 2, 1971, 971-972) und Dutrillaux et al. (C R Acad. Sci. Paris, 272, 1971, 3638-3640) entwickelt wurden. Mit diesen Verfahren kann jedoch die Identität einzelner chromosomaler Banden nicht in jedem Fall definiert werden, da alle Banden von allen Chromosomen lediglich entweder hell oder dunkel erscheinen. Dies erweist sich als ein wesentlicher Nachteil, da Chromosomen von Zelle zu Zelle und von Gewebe zu Gewebe morphologisch sehr unterschiedlich sein können und u.U. Translokationen (zum Beispiel bei Tumoren) aufweisen, deren Erkennung für die zu untersuchende Person von besonderer Bedeutung sein kann. Dies gilt beispielsweise für die Realisierung von Kinderwunsch bei balancierten Translokationen ("Chromosomenstückaustauschen") eines Elternteils, für die Erkennung der Ursache von Fehlbildungen bei Kindern mit und ohne geistige Retardierung und für die Diagnostik von Leukämien und anderer Tumoren, die häufig spezifische chromosomale Veränderungen von diagnostischer und therapeutischer Bedeutung aufweisen.

Als Vorschlag zur Lösung dieser Problematik wurde von Pinkel et al. (Proc. Natl.

Acad. Sci. USA 83, 1986, 2934-2938) die Fluoreszenz-in-situ-Hybridisierung (FISH) erstmals für die Routine in praktikabler Form beschrieben. Mit diesem Verfahren können heute durch den Einsatz chromosomenspezifischer DNA-Banken alle menschlichen Chromosomen einer Metaphase in jeweils unterschiedlichen Farben dargestellt werden (chromosome painting, 24-colour-FISH, Schröck et al., Science 273, 1996, 496-497; Speicher et al. Nature Genet. 12, 1996, 368-375), und durch den Einsatz von Vektoren, beispielsweise Cosmiden, Pac's oder YAC's, die unterschiedliche Mengen an menschlicher DNA enthalten können, spezifische chromosomale Regionen erneut über Vielfarbentechniken mittels FISH in ihrer Integrität überprüft werden. Auch Teile von Genen und repetitive DNA-Elemente lassen sich über diesen Weg auf ihre chromosomale Lokalisation hin und auf ihr Vorhandensein bzw. Fehlen nachweisen. Eine mehrfarbige ("multicolor"-) Darstellung um chromosomalen Abschnitten auf Bandenniveau ist bisher jedoch nicht möglich.

Druckschrift D1 (Müller, S. et al, 1997) beschreibt ein Verfahren, worin zwei mit verschiedenen Fluorochromen (hier grün und rot) markierte Sätze von subregionalen DNA-Sonden (DNA-Pools) auf menschlichen Metaphase-Chromosomen *in situ* hybridisiert werden. Es entsteht ein reproduzierbares Bändermuster aus roten, grünen und gelben (an Stellen, an die beide Pools binden) Banden (vgl. beispielsweise "Abstract").

Druckschrift D2 (WO 97/40191) beschreibt ein Spektralbildverfahren zum Nachweis und zur Analyse von verschiedenen Fluorochromen bei der Chromosomenfarbbänderung. Die Markierung der Chromosomen erfolgt dabei durch *in situ*-Hybridisierung mit Chromosomenfragmenten, die mit verschiedenen Fluorochromen oder Kombinationen von Fluorochromen markiert sind. Danach werden für die Fluorochrome, die über die Chromosomenfragmente an das Chromosom gebunden sind, vollständige Spektren gemessen, wobei sich für jedes Chromosom ein definiertes Farbbandenmuster ergibt (vgl. beispielsweise "Abstract" und Anspruch 1).

Somit liegt der vorliegenden Erfindung die Aufgabe zugrunde, ein neues bzw. verbessertes Verfahren zum Nachweis von insbesondere Änderungen in chromosomaler DNA bereitzustellen, das eine mehrfarbige Darstellung auf Bandenniveau ermöglichen soll.

Diese Aufgabe wird durch die in den Ansprüchen gekennzeichneten Ausführungsformen der vorliegenden Erfindung gelöst.

Insbesondere wird ein Verfahren zum Nachweis von Änderungen in Biopolymeren als Zielmoleküle unter Verwendung von zwei oder mehreren unterschiedlichen Sätzen von markierten Detektormolekülen bereitgestellt, wobei jeweils mindestens zwei Sätze für einen bestimmten Bereich in den Zielmolekülen spezifisch sind und die Markierungen der jeweiligen Detektormoleküle diese für einen bestimmten Bereich in den Zielmolekülen spezifischen Sätze unterschiedlich sind, umfassend die Schritte
(a) Durchführen von Bindungsreaktionen zwischen den Detektormolekülen der unterschiedlichen Sätze und den Zielmolekülen, wobei die jeweiligen markierten Detektormoleküle von mindestens zwei Sätzen derart an einen bestimmten Bereich der Zielmoleküle binden, daß sich die unterschiedlichen Markierungen der Detektormoleküle überlappen, und
(b) Aufzeichnen der Intensitäten bzw. Intensitätsverhältnisse der unterschiedlichen Markierungen unter Verwendung einer Abtastvorrichtung, bei welcher die Intensitäten bzw. Intensitätsverhältnisse der Markierungen in den Bereichen von überlappenden und nicht-überlappenden Markierungen der jeweiligen Detektormoleküle in Längsrichtung der Zielmoleküle aufgezeichnet werden, und
(c) Auswerten der aufgezeichneten Intensitäten bzw. Intensitätsverhältnisse, wobei das Zielmolekül in mehrere kleine Abschnitte unterteilt wird und über ein geeignetes Rechenprogramm jedem dieser Abschnitte auf der Basis der relativen Intensitätverhältnisse eine Falschfarbe zugeordnet wird.

Der Begriff "Biopolymere als Zielmoleküle" bedeutet DNA, vorzugsweise chromosomale DNA, RNA oder Polypeptide. Die Zielmoleküle können vor Durchführung des erfindungsgemäßen Verfahrens insbesondere vor Schritt (a) entsprechend angeordnet bzw. immobilisiert werden, beispielsweise durch gelelektrophoretische Auftrennung in einer geeigneten Matrix oder Fixierung bzw. Anordnung von beispielsweise Metaphase-Chromosomen oder Interphase-Kernen auf einen geeigneten Träger.

Der Begriff "markierte Detektormoleküle" bedeutet Nukleinsäuren oder Antikörper, die jeweils mindestens eine Markierung aufweisen. Die Antikörper können polyklonal oder monoklonal vorliegen. Die Begriffe "Nukleinsäure" bzw. "Nukleinsäuresequenz" bzw. "Nukleinsäuresonden" bedeuten native, halbsynthetische oder modifizierte Nukleinsäuremoleküle aus Desoxyribonukleotiden und/oder Ribonukleotiden und/oder modifizierten Nukleotiden wie Aminonukleotiden oder [α-S]-Triphosphatnukleotiden. In einer bevorzugten Ausführungsform der vorliegenden Erfindung stammen die Nukleinsäuren von chromosomaler DNA aus beispielsweise Säugern wie homo sapiens sapiens. Die chromosomale DNA als Detektormoleküle liegt in Vektoren, z.B. Cosmide oder YAC's vor, oder stammt aus chromosomalen bzw. Chromosomenregion-spezifischen DNA-Banken, die beispielsweise über Mikrodissektionsverfahren oder Laser aktivierte flußcytometrische Sortierung von spezifischen Chromosomen und, wenn erforderlich, nachfolgender Amplifikation durch beispielsweise DOP-PCR erhalten werden können.

Der Begriff "Markierung" bedeutet geeignete, direkt oder indirekt nachweisbare Atome oder Moleküle, die in die Detektormoleküle eingebaut oder damit verbunden sind. Geeignete Markierungen sind beispielsweise solche, die an Nukleotide gekoppelte Fluoreszenzfarbstoffe und/oder beispielsweise Biotin und/oder Digoxigenin und/oder mit radioaktiven Isotopen markierte Nukleotide umfassen. In einer bevorzugten Ausführungsform ist die Markierungsverbindung ein Fluoreszenzfarbstoff mit einer zur Selektion kleiner Substanzmengen ausreichendem Unterschied im Fluoreszenzverhalten der Emissionsspektren, wie z.B. Cumarine und Rodamine und/oder der Fluoreszenzlebensdauer wie, z.B. Fluoreszenzisothiocyanate und Europium-Chelat-markierte und/oder Porphirin-markierte Avidine.

Der Begriff "Bindungsreaktion" bedeutet eine Hybridisierung, vorzugsweise eine *in-situ*-Hybridisierung, oder eine Antigen/Antikörper-Reaktion, abhängig von der Wahl der Detektormoleküle und/oder der Zielmoleküle. Der Begriff "*in-situ*-Hybridisierung" bedeutet die Anlagerung einer synthetisch erzeugten und mit biologischen, physikalischen oder chemischen Markierungen zur Detektion versehenen DNA/RNA-Sonde als Detektormolekül an native in der Natur vorkommende DNA/RNA-Sequenzen, wobei die Anlagerung durch Denaturierung und Renaturierung der entsprechenden Nukleinsäuren erreicht wird. Selbstverständlich enthalten diese DNA/RNA-Sonden mindestens einen zur Hybridisierung an eine DNA/RNA-Sequenz des Zielmoleküls, wie ein Chromosom, befähigten Sequenzabschnitt. Dieser Sequenzabschnitt weist einen spezifischen, einzeln vorliegenden, vorzugsweise 100 bis 1.000 Basenpaare langen Sequenzbereich des Detektormoleküls auf, der sich an einen komplementären Bereich des Zielmoleküls bei einer geeigneten Temperatur, vorzugsweise bei 50°C oder weniger, und bei einer geeigneten Salzkonzentration, die vorzugsweise 50-300 mmol/l einwertige Ionen und 0-10 mmol/l zweiwertige Ionen enthält, unter Ausbildung von Wasserstoffbrücken anlagert. Die Bindungsreaktion der jeweiligen Sätze von markierten Detektormolekülen kann gleichzeitig oder aufeinanderfolgend durchgeführt werden.

Der Ausdruck "Satz von Detektormolekülen" bedeutet Detektormoleküle, die für einen bestimmten Bereich der Zielmoleküle spezifisch sind. Dieser Satz von Detektormolekülen kann beispielsweise in Vektoren vorliegende chromosomale DNA oder eine chromosomenspezifische DNA-Bank sein. Die Markierung der Detektormoleküle im Satz können gleich oder unterschiedlich sein, beispielsweise drei unterschiedliche Markierungen enthalten.

Der Ausdruck "mindestens zwei oder mehrere unterschiedliche Sätze von markierten Detektormolekülen" bedeutet das Vorliegen von mindestens einem Paar von unterschiedlichen Sätzen, wobei die Sätze dieses Paars in einem bestimmten Bereich bzw. Region der Zielmoleküle derart binden, daß sich mindestens die unterschiedlichen Markierungen der jeweiligen Detektormoleküle, vorzugsweise die Bindungsstellen der jeweiligen Detektormoleküle dieser unterschiedlichen Sätze überlappen. Diese erfindungsgemäße Eigenschaft von einem Paar unterschiedlicher Sätze bedeutet, daß die jeweiligen Detektormoleküle in den unterschiedlichen Sätzen eines Paars, welches aus diesem bestimmten Bereich der Zielmoleküle überlappend hergestellt bzw. gewonnen werden, als Standard bzw. zur vergleichenden Untersuchung mit entsprechend aufgearbeiteten Proben von Patienten verwendet werden können. In einer erfindungsgemäßen Ausführungsform sind die Detektormoleküle eines Satzes vorzugsweise derart gestaltet, daß nach der Hybridisierung die Detektormoleküle in kontinuierlich veränderter Konzentration, vorzugsweise in Art einer Gaußschen Verteilung, in Längsrichtung an die Zielmoleküle, beispielsweise Chromosomen, gebunden sind.

Die in Schritt (b) des erfindungsgemäßen Verfahrens gekennzeichnete qualitative und quantitative Auswertung der in Schritt (a) erhaltenen Bindungen über die unterschiedlichen Markierungen der Detektormoleküle kann unter Verwendung einer Abtasteinrichtung bzw. einer Vorrichtung zum gerichteten Abtasten, beispielsweise entlang bzw. in Längsrichtung des zu untersuchenden Chromosoms, verwendet werden. Eine derartige Abtasteinrichtung ist beispielsweise ein Fluoreszenzmikroskop. Über die physikalischen und/oder chemischen und/oder biologischen Markierungen der Detektormoleküle, die sich an die gewünschten Zielmoleküle angelagert haben, können durch die Abtasteinrichtung bilderzeugende Signale über eine Bildverarbeitungseinheit, beispielsweise eine CCD-Kamera, aufgenommen werden, welche computergestützt die einzelnen Signale der unterschiedlichen Markierungen in geeigneter Weise verarbeitet. Mit dieser an die Abtastvorrichtung gekoppelten Bildverarbeitungseinheit können die Intensitäten bzw. die Intensitätsverhältnisse der unterschiedlichen Markierungen in den Bereichen von überlappenden und nicht-überlappenden Markierungen der jeweiligen Detektormoleküle vorzugsweise in Längsrichtung der Zielmoleküle, insbesondere von fixierten Metaphase-Chromosomen, aufgezeichnet und qualitativ sowie quantitativ ausgewertet werden.

Ein weiterer Gegenstand der vorliegenden Erfindung ist eine automatische Korrektur durch Hinzufügen einer lokalisierten DNA-Sonde.

Bei der Aufnahme von mit verschiedenen Fluorochromen markierten chromosomenregion-spezifischen Proben, wie sie für die Methode des Multi Color Banding eingesetzt werden, wird eine Monochrom-CCD Kamera in Kombination mit spezifischen Fluoreszenzfiltern verwendet. Die Signale der einzelnen Fluorochrome werden nacheinander als Einzelbilder aufgenommen und anschließend zu einem Farbbild zusammengesetzt. Eine Verschiebung der Lage der Einzelbilder gegeneinander aufgrund optischer Einflüsse der Filter (unterschiedliche Keilfehler, Parallelversatz durch geringfügige Verkippung im Strahlengang) ist dabei nicht auszuschließen. Eine interaktive oder automatische Korrektur, etwa durch eine Korrelation der Einzelbilder, ist nicht mit der erforderlichen Präzision möglich, da die allenfalls teilweise überlappenden Sonden keine gemeinsamen Strukturen aufweisen, die für eine nachträgliche Überlagerung genutzt werden können. Jede geringfügige Verschiebung führt bei der Auswertung der Intensitätsverhältnisse zu Artefakten im Bänderungsmuster.

Die automatische Korrekur wird durch Beifügen einer lokalisierten DNA-Sonde ermöglicht, die mit allen im erfindungsgemäßen Verfahren verwendeten Fluorochromen gleichzeitig markiert ist. Damit steht eine in allen Einzelbildern identische Struktur zur automatischen Lagekorrektur zur Verfügung. Die Lagekorrektur kann z. B. über eine Ermittlung des Intensitätsschwerpunkts der Sonde in jedem Einzelbild und anschließend relativer Verschiebung der Einzelbilder so erfolgen, daß die Schwerpunkte der Einzelbilder auf die gleiche Position zu liegen kommen.

Durch den Einsatz zweier verschiedener Sonden können auch Verdrehungen der Bilder gegeneinander ermittelt und korrigiert werden.

Der Einsatz von noch mehr Sonden ermöglicht grundsätzlich die Korrektur von komplexeren Lagetransformationen als Translation und Rotation, wie z. B. Maßstabsveränderungen.

Ferner kann es in einer weiteren bevorzugten Ausführungsform der vorliegenden Erfindung von Vorteil sein, Kalibriersonden (DNA-Sonden oder fluoreszierende Partikel) bekannter Intensität beizufügen, die zur Normierung der Intensitäten der auszuwertenden Fluoreszenzsignale dienen können.

Ferner kann es in einer weiteren bevorzugten Ausführungsform der vorliegenden Erfindung von Vorteil sein, DNA-Sonden beizufügen, deren genaue Lokalisation innerhalb des Genoms bekannt sind, und die dazu verwendet werden können, den Bezug zwischen Farbbanden und den ISCN-Banden herzustellen.

Die Figuren zeigen:

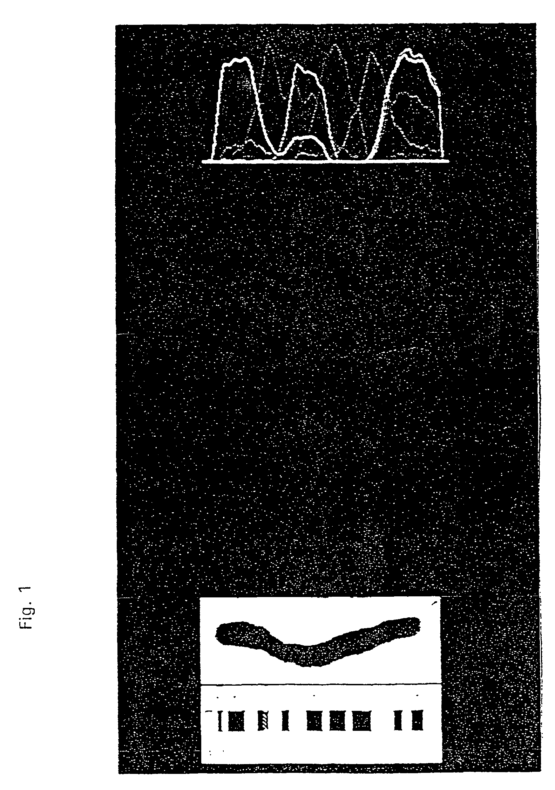

- Fig. 1: ist eine photographische Darstellung zur qualitativen und quantitativen Auswertung der Lokalisation von Region-spezifischen Färbungen in Chromosom 5. Im oberen Teil dieser Figur ist die Verteilung der markierten Detektormoleküle in Längsrichtung des Chromosoms sowie Intensitäten der unterschiedlichen Markierungen der Detektormoleküle graphisch dargestellt.

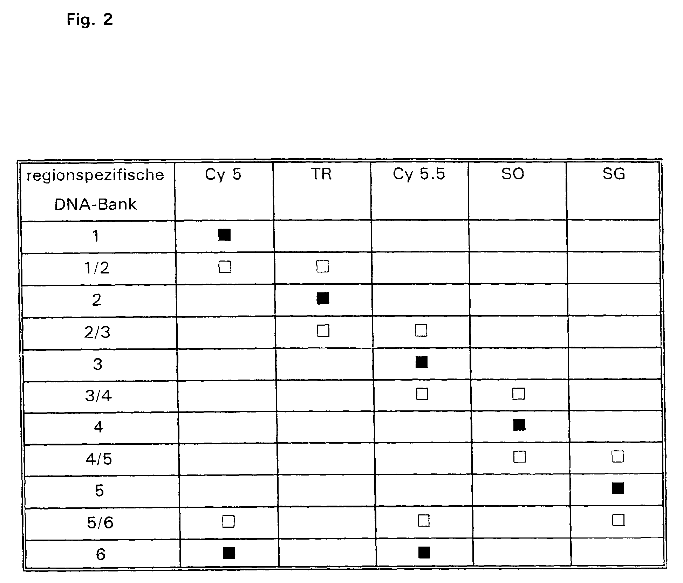

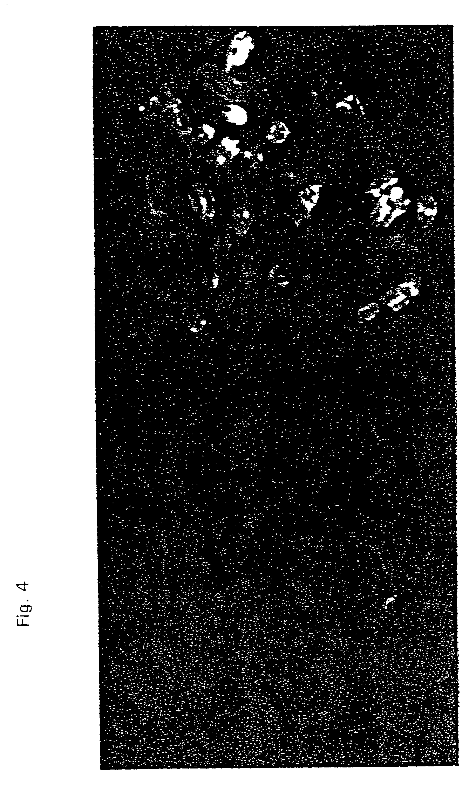

- Fig. 2: ist eine tabellarische Darstellung des Markierungsmusters des in Fig. 1 dargestellten Region-spezifischen Chromosomenabschnitts von Chromosom 5. Cy5, TR(Texas Red), Cy5.5, SO (Spectrum Orange), SG (Spectrum Green) sind die verschiedenen FluoreszenzFarbstoffe, die zum Markieren der einzelnen regionspezifischen DNA-Banken verwendet wurden. Die Zuordnung ist durch eine ausgefülltes Quadrat (■) gekennzeichnet. Die durch die Überlappung der DNA-Banken sich ergebende Markierungen in den entsprechenden Bereichen ist durch ein leeres Quadrat (□) kenntlich gemacht.
- Fig. 3: zeigt jeweils die homologen normalen Chromosomen 5 aus zwei verschiedenen Metaphaseplatten mit mehrfarbiger Bänderung. Die Darstellung macht deutlich, daß das Bänderungsmuster auf den homologen Chromosomen identisch ist und auch von Metaphaseplatte zu Metaphaseplatte reproduzierbar ist.
- Fig. 4: zeigt eine photographische Darstellung einer Vielfarben-FISH einer Metaphaseplatte mit komplexen chromosomalen Abänderungen.
- Fig. 5: zeigt Chromosomen 5 in einem Fall mit akuter myeloischer Leukämie. Auf der rechten Seite ist jeweils das normale Chromosom 5 dargestellt, das Chromosom auf der linken Seite zeigt eine interstitielle Deletion im langen Arm.

Das folgende Beispiel erläutert die Erfindung.

### Beispiel

Für das Vielfarben-Bandenmuster von Chromosom 5 wurden insgesamt 7 überlappende Chromosomenregion-spezifische Mikrosezierungsbanken hergestellt (Meltzer et al., Nature Genet. 1, 1992, 24-28). Der p-Arm von Chromosom 5 wurde hierfür in zwei, der q-Arm in vier Regionen unterteilt. Pro Chromosomenregion wurden jeweils 8-10 Fragmente mit einer fein ausgezogenen Glasnadel vom Objektträger unter mikroskopischer Sicht isoliert (Senger et al., Hum. Genet. 84, 1990, 507-511). Die so gewonnene DNA wurde über eine DOP-PCR (degenerate oligonucleotide polymerase chain reaction, Telenius et al., Genomics 13, 1992, 718-725; Zhang et al., Blood 81, 1993, 3365-3371) amplifiziert. In einer Folgereaktion wurden diese Chromosomenregion-spezifischen DNA-Banken teilweise direkt mit Fluorochromen, die an Nukleotide gekoppelt vorliegen, markiert (z.B. Spectrum Orange-dUTP, Spectrum Green-dUTP, beide Vysis, und Texas Red-dUTP, Molecular Probe). Zum anderen Teil wurden DNA-Banken mit Nukleotiden markiert, die mit Haptenen (z.B. Biotin-dUTP und DigoxigenindUTP, Boehringer, Mannheim) gekoppelt sind. Nach erfolgter Hybridisierung können Haptene mit geeigneten Detektionsreagenzien (z.B. Avidin-Cy5, Amersham und Anti-Digoxigenin !gG, Boehringer, Mannheim, der an Cy5.5 gekoppelt ist, Mab-labeling kit, Amersham) detektiert werden.

Die Hybridisierung, Waschschritte und Detektion erfolgen nach Standardprotokollen (Senger et al., Cytogenet. Cell. Genet. 64, 1993, 49-53).

Die Analyse wird z.B. mit einem Fluoreszenzmikroskop durchgeführt, das mit geeigneten Filtersätzen ausgestattet ist. Von jedem Farbkanal werden separat Bilder aufgenommen, die anschließend mit einem Computer weiterbearbeitet werden können.

Ein charakteristisches Merkmal der Teil-"painting "-Sonden, die durch Mikrosezierung gewonnen werden, ist ein sich kontinuierlich abschwächendes Fluoreszenzsignal in den Randbereichen. Durch gleichzeitiges Überlappen der Sonden und damit der Fluoreszenzsignale zweier benachbarter Teil-"painting"-Sonden kommt es zu einem sich kontinuierlich verändernden Verhältnis der Fluoreszenzintensitäten entlang von Chromosom 5. Wird ein so gefärbtes Chromosom in mehrere (20-25) kleine Abschnitte unterteilt, so kann über ein geeignetes Rechenprogramm jedem dieser Sektoren auf der Basis der relativen Fluoreszenzintensitäten aller verwendeten Fluorchrome eine Falschfarbe zugeordnet werden. Durch diese Zuordnung kommt es zu einem farblichen Bandenmuster entlang eines Chromosoms, in diesem Fall von Chromosom 5. Bei allen weiteren Hybridisierungen mit dem selben Probenset kann die gleiche Kombination von Fluoreszenzverhältnissen und Falschfarben verwendet werden.

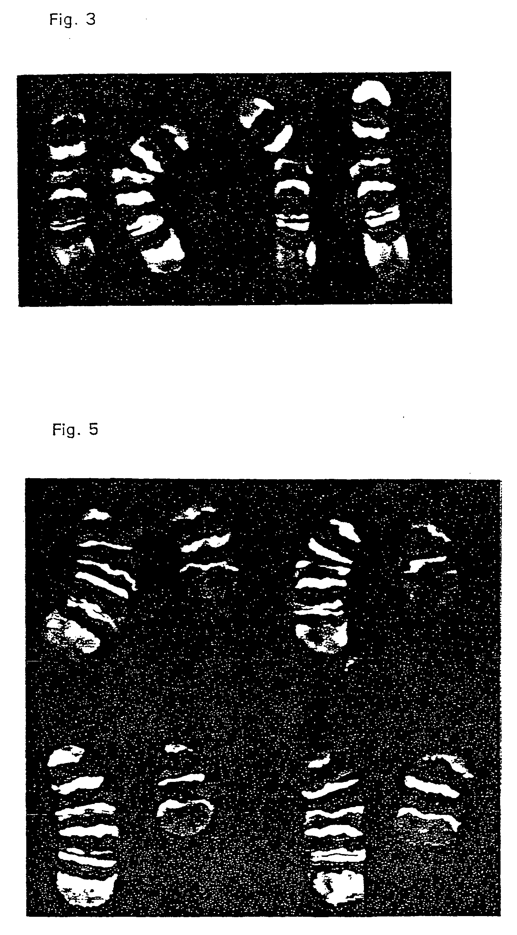

Da die Hybridisierung sich ausreichend konstant verhält, ist auch das Bandenmuster entsprechend reproduzierbar (Figur 3). Ein Verlust des Auflösungsvermögens bei kürzeren Chromosomen, wie es von bislang üblichen Bänderungsverfahren (z.B. GTG-Bänderung) bekannt ist, wird hierbei nicht beobachtet. Für das Chromosom 5 wird ein reproduzierbares Muster von mindestens 25 Banden erreicht. Dies entspricht einem Bandenniveau von ca. 550 Banden pro haploiden Chromosomensatz.

Mit Hilfe dieses Verfahrens ist es möglich Veränderungen an Chromosomen nachzuweisen unabhängig von ihrem Kondensationszustand. Dies ist vor allem auch in der Tumorzytogenetik von Bedeutung. Tumorchromosomen zeigen oft eine geringe Auflösung des Bandenmusters, wodurch ein Erkennen von chromosomalen Veränderungen wesentlich erschwert ist. Es ist daher anzunehmen, daß bislang unbekannte zytogenetische Veränderung in Tumoren vorliegen, die möglicherweise einen wichtigen Prognosefaktor darstellen und daher z.B. für eine risikoangepaßte Therapie von Bedeutung sein könnten. Erfindungsgemäß können auch an Tumorchromosomen nach Hybridisierung mit dem oben näher beschriebenen Probenset für das Chromosom 5 mindestens 25 Banden erreicht werden (Figur 5).

## Patentansprüche

1. Verfahren zum Nachweis von Änderungen in Biopolymeren als Zielmoleküle unter Verwendung von zwei oder mehreren unterschiedlichen Sätzen von markierten Detektormolekülen, wobei jeweils mindestens zwei Sätze für einen bestimmten Bereich in den Zielmolekülen spezifisch sind und die Markierungen der jeweiligen Detektormoleküle dieser für einen bestimmten Bereich in den Zielmolekülen spezifischen Sätze unterschiedlich sind, umfassend die Schritte
(a) Durchführen von Bindungsreaktionen zwischen den Detektormolekülen der unterschiedlichen Sätze und den Zielmolekülen, wobei die jeweiligen markierten Detektormoleküle von mindestens zwei Sätzen derart an einen bestimmten Bereich der Zielmoleküle binden, daß sich die unterschiedlichen Markierungen der jeweiligen Detektormoleküle überlappen,
(b) Aufzeichnen der Intensitäten bzw. Intensitätsverhältnisse der unterschiedlichen Markierungen unter Verwendung einer Abtastvorrichtung, bei welcher die Intensitäten bzw. Intensitätsverhältnisse der Markierungen in den Bereichen von überlappenden und nicht-überlappenden Markierungen der jeweiligen Detektormoleküle in Längsrichtung der Zielmoleküle aufgezeichnet werden, und
(c) Auswerten der aufgezeichneten Intensitäten bzw. Intensitätsverhältnisse, wobei das Zielmolekül in mehrere kleine Abschnitte unterteilt wird und über ein geeignetes Rechenprogramm jedem dieser Abschnitte auf der Basis der relativen Intensitätverhältnisse eine Falschfarbe zugeordnet wird.

2. Verfahren nach Anspruch 1, worin die Zielmoleküle vor Schritt (a) immobilisiert werden.

3. Verfahren nach Anspruch 2, worin die Zielmoleküle bzw. das Zielmolekül auf einem Träger oder in einer Matrix angeordnet sind.

4. Verfahren nach einem der Ansprüche 1 bis 3, worin die Bindungsreaktionen der jeweiligen Sätze von markierten Detektormolekülen gleichzeitig oder aufeinanderfolgend durchgeführt werden.

5. Verfahren nach einem der Ansprüche 1 bis 4, wobei die Bindungsreaktion in Schritt (a) eine Hybridisierung oder eine Antigen/Antikörper-Reaktion ist.

6. Verfahren nach Anspruch 5, wobei die Hybridisierung eine *in situ* Hybridisierung ist.

7. Verfahren nach einem der Ansprüche 1 bis 6, wobei die Zielmoleküle Nukleinsäuren oder Polypeptide sind.

8. Verfahren nach Anspruch 7, wobei die Nukleinsäuren aus DNA und RNA ausgewählt sind.

9. Verfahren nach Anspruch 7 oder 8, wobei die Nukleinsäuren chromosomale DNA sind.

10. Verfahren nach einem der Ansprüche 1 bis 9, wobei die markierten Detektormoleküle aus Nukleinsäuren oder Antikörper ausgewählt sind.

11. Verfahren nach Anspruch 10, wobei die jeweiligen Sätze von Nukleinsäuren von unterschiedlichen chromosomenregion-spezifischen DNA-Banken stammen.

12. Verfahren nach Anspruch 10 oder 11, wobei jeder Satz von Detektormolekülen ein oder mehrere unterschiedliche Markierungen enthält.

13. Verfahren nach Anspruch 12, wobei die Markierung einen Fluoreszenzfarbstoff umfaßt.

14. Verfahren nach einem der Ansprüche 1 bis 13, worin mindestens eine mit mindestens zwei der verwendeten N Fluorochrome markierte DNA-Sonde zur Ermittlung einer optisch verursachten relativen Verschiebung der Bildinformation der Fluorochrome und zu ihrer Lagekorrektur beigefügt ist.

15. Verfahren nach Anspruch 14, worin mindestens eine mit allen N verwendeten Fluorochromen markierte DNA-Sonde zur gleichzeitigen Lagekorrektur aller Fluorochrome beigefügt ist.

16. Verfahren nach Anspruch 14, worin N-1 DNA-Sonden mit je zwei geeigneten Fluorochromen markiert sind und die Lagekorrektur paarweise durchgeführt wird.

17. Verfahren nach einem der Ansprüche 14 bis 16, worin durch Verwendung einer ausreichenden Zahl von DNA-Sonden Lagetransformationen mit mehr Freiheitsgraden korrigiert werden.

18. Verfahren nach einem der Ansprüche 14 bis 17, worin die Ermittlung der relativen Verschiebungen und die Lagekorrektur interaktiv erfolgen.

19. Verfahren nach einem der Ansprüche 14 bis 18, worin die Ermittlung der relativen Verschiebungen und die Lagekorrektur automatisch erfolgen.

20. Verfahren nach einem der Ansprüche 14 bis 19, worin Kalibriersonden bekannter bzw. reproduzierbarer konstanter Intensität zur Normierung der Signalintensitäten beigefügt werden.

21. Verfahren nach Anspruch 20, wobei die Kalibriersonden fluoreszenzmarkierte DNA-Sonden sind.

22. Verfahren nach Anspruch 20, wobei die Kalibriersonden fluoreszenzmarkierte Partikel sind.

23. Verfahren nach einem der Ansprüche 14 bis 22, worin die Kalibriersonden gleichzeitig zur Lagekorrektur verwendet werden.

## Claims

1. Method for detecting changes in biopolymers as target molecules using two or more different sets of labelled detector molecules, with in each case at least two sets being specific for a particular region in the target molecules and the labels of the respective detector molecules of these sets which are specific for a particular region in the target molecules being different, comprising the steps of
(a) carrying out binding reactions between the detector molecules of the different sets and the target molecules, with the respective labelled detector molecules of at least two sets binding to a particular region of the target molecules such that the different labels of the respective detector molecules overlap,
(b) recording the intensities or intensity ratios of the different labels using a sensing device in which the intensities or intensity ratios of the labels in the regions of overlapping and non-overlapping labels of the respective detector molecules are recorded in the longitudinal direction of the target molecules, and
(c) analysing the recorded intensities or intensity ratios, with the target molecule being subdivided into several small sections and a suitable computing program being used to assign a false colour to each of these sections on the basis of the relative intensity ratios.

2. Method according to Claim 1, wherein the target molecules are immobilized prior to step (a).

3. Method according to Claim 2, wherein the target molecules or the target molecule is/are arranged on a support or in a matrix.

4. Method according to one of Claims 1 to 3, wherein the binding reactions of the respective sets of labelled detector molecules are carried out simultaneously or consecutively.

5. Method according to one of Claims 1 to 4, wherein the binding reaction in step (a) is a hybridization or an antigen/antibody reaction.

6. Method according to Claim 5, wherein the hybridization is an *in-situ* hybridization.

7. Method according to one of Claims 1 to 6, wherein the target molecules are nucleic acids or polypeptides.

8. Method according to Claim 7, wherein the nucleic acids are selected from DNA and RNA.

9. Method according to Claim 7 or 8, wherein the nucleic acids are chromosomal DNA.

10. Method according to one of Claims 1 to 9, wherein the labelled detector molecules are selected from nucleic acids or antibodies.

11. Method according to Claim 10, wherein the respective sets of nucleic acids are derived from different chromosome region-specific DNA libraries.

12. Method according to Claim 10 or 11, wherein each set of detector molecules contains one or more different labels.

13. Method according to Claim 12, wherein the label comprises a fluorescent dye.

14. Method according to one of Claims 1 to 13, wherein at least one DNA probe which is labelled with at least two of the N fluorochromes employed is included for ascertaining an optically induced relative displacement of the image information of the fluorochromes and for correcting their positions.

15. Method according to Claim 14, wherein at least one DNA probe which is labelled with all the N fluorochromes employed is included for simultaneously correcting the positions of all the fluorochromes.

16. Method according to Claim 14, wherein N-1 DNA probes are labelled with in each case two suitable fluorochromes and the position correction is carried out in pairs.

17. Method according to one of Claims 14 to 16, wherein position transformations are corrected with several degrees of freedom by using a sufficient number of DNA probes.

18. Method according to one of Claims 14 to 17, wherein the relative displacements and the position correction are ascertained interactively.

19. Method according to one of Claims 14 to 18, wherein the relative displacements and the position correction are ascertained automatically.

20. Method according to one of Claims 14 to 19, wherein calibration probes of known or reproducible constant intensity are included for standardizing the signal intensities.

21. Method according to Claim 20, wherein the calibration probes are fluorescence-labelled DNA probes.

22. Method according to Claim 20, wherein the calibration probes are fluorescence-labelled particles.

23. Method according to one of Claims 14 to 22, wherein the calibration probes are simultaneously used for position correction.

## Revendications

1. Procédé de détection des modifications de biopolymères comme molécules cibles par utilisation de deux ou plusieurs lignes de molécules détectrices marquées, dans lequel au moins deux lignes sont spécifiques à une zone déterminée dans les molécules cibles, et dans lequel les marquages des molécules détectrices respectives de ces lignes spécifiques à une zone déterminée dans les molécules cibles sont différents, incluant les étapes de:
(a) réalisation de réactions de liaison entre les molécules détectrices des différentes lignes et les molécules cibles, dans lequel les molécules détectrices marquées respectives d'au moins deux lignes se lient à une zone déterminée des molécules cibles de telle sorte que les différents marquages des molécules détectrices respectives se chevauchent,
(b) enregistrement des intensités ou des rapports d'intensité des différents marquages par utilisation d'un dispositif de balayage par lequel sont enregistrés les intensités ou respectivement les rapports d'intensité des marquages dans les zones de marquage chevauchant et non-chevauchant des molécules détectrices respectives suivant la longueur des molécules cibles, et
(c) interprétation des intensités ou respectivement des rapports d'intensités enregistrés, dans lequel la molécule cible est subdivisée en plusieurs petites sections et se voit octroyer pour chacune de ces sections une fausse couleur sur la base des rapports d'intensité relatifs par un programme de calcul adéquat.

2. Procédé selon la revendication 1, dans lequel les molécules cibles sont immobilisées avant l'étape (a).

3. Procédé selon la revendication 2, dans lequel les molécules cibles ou respectivement la molécule cible sont rangées sur un support ou dans une matrice.

4. Procédé selon une des revendications 1 à 3 dans lequel les réactions de liaison des lignes respectives de molécules détectrices marquées sont effectuées simultanément ou les unes après les autres.

5. Procédé selon une des revendications 1 à 4 dans lequel la réaction de liaison à l'étape (a) est une hybridation ou une réaction anticorps-antigène.

6. Procédé selon la revendication 5 dans lequel l'hybridation est une hybridation *in situ.*

7. Procédé selon une des revendications 1 à 6 dans lequel les molécules cibles sont des acides nucléiques ou des polypeptides.

8. Procédé selon la revendication 7 dans lequel les acides nucléiques sont choisis à partir d'ADN ou d'ARN.

9. Procédé selon la revendication 7 ou 8 dans lequel les acides nucléiques sont de l'ADN chromosomique.

10. Procédé selon une des revendications 1 à 9 dans lequel les molécules détectrices marquées sont choisies à partir d'acides nucléiques ou d'anticorps.

11. Procédé selon la revendication 10 dans lequel les lignes respectives d'acides nucléiques proviennent de différentes banques d'ADN spécifiques de régions chromosomiques données.

12. Procédé selon la revendication 10 ou 11 dans lequel chaque ligne de molécules détectrices contient un ou plusieurs marquages différents.

13. Procédé selon la revendication 12 dans lequel le marquage comprend un colorant fluorescent.

14. Procédé selon une des revendications 1 à 13 dans lequel au moins une sonde ADN marquée avec au moins deux des fluorochromes N utilisés est jointe pour déterminer un décalage optique relatif de l'information obtenue par la figuration graphique des fluorochromes et pour corriger leur position.

15. Procédé selon la revendication 14 dans lequel au moins une des sondes ADN marquée avec tous les fluorochromes N utilisés est jointe pour la correction simultanée de la position de tous les fluorochromes.

16. Procédé selon la revendication 14 dans lequel des sondes ADN N1 sont marquées avec chacune deux fluorochromes adéquats et dans lequel la correction de position est effectuée par paires.

17. Procédé selon une des revendications 14 à 16 dans lequel les transformations de position sont corrigées avec davantage de degrés de liberté par l'utilisation d'un nombre suffisant de sondes ADN.

18. Procédé selon une des revendications 14 à 17 dans lequel la recherche des décalages relatifs et la correction de position se font de façon interactive.

19. Procédé selon une des revendications 14 à 18 dans lequel la recherche des décalages relatifs et la correction de position se font de façon automatique.

20. Procédé selon une des revendications 14 à 19 dans lequel sont jointes des sondes d'étalonnage d'intensité constante connue ou reproductible pour normaliser les intensités des signaux.

21. Procédé selon la revendication 20 dans lequel les sondes d'étalonnage sont des sondes ADN marquées par fluorescence.

22. Procédé selon la revendication 20 dans lequel les sondes d'étalonnage sont des particules marquées par fluorescence.

23. Procédé selon une des revendications 14 à 22 dans lequel les sondes d'étalonnage sont utilisées simultanément pour la correction de position.
